# EUROPEAN PATENT SPECIFICATION

(11) **EP 1 909 561 B1**
(45) Date of publication and mention of the grant of the patent: **07.04.2010**
(21) Application number: 06777668.2
(22) Date of filing: 10.07.2006
(51) Int. Cl.: A01K 29/00, A01K 1/03, A01K 67/02

(54) **A METHOD FOR PROVIDING AND ANALYZING AN ANIMAL POPULATION HAVING AN ESSENTIALLY IDENTICAL METABOLOME**
VERFAHREN ZUR BEREITSTELLUNG UND ANALYSE EINER TIERPOPULATION MIT IM WESENTLICHEM IDENTISCHEM METABOLOM
PROCÉDÉ DE FOURNITURE ET D'ANALYSE D'UNE POPULATION ANIMALE AYANT UN MÉTABOLOME ESSENTIELLEMENT IDENTIQUE

(30) Priority: 25.07.2005 EP 05016064; 26.08.2005 EP 05018611
(43) Date of publication of application: 16.04.2008
(73) Proprietor: BASF SE, 67056 Ludwigshafen (DE)
(72) Inventor: RAVENZWAAY, Bennard van, 67122 Altrip (DE); COELHO PALERMO CUNHA, Georgia, 67117 Limburgerhof (DE); MELLERT, Werner, 67454 Hassloch (DE); LOOSER, Ralf, 13158 Berlin (DE); WALK, Tilmann B., 14532 Kleinmachnow (DE); WIEMER, Jan C., 13503 Berlin (DE); HAAKE, Volker, 13503 Berlin (DE)
(86) International application number: PCT/EP2006/064053
(87) International publication number: WO 2007/014825

(56) References cited:
- EP-A- 0 375 030
- EP-A- 1 454 523
- WO-A-02/067667
- US-A- 3 464 388
- US-A- 5 163 380

## Description

The present invention relates to a method for providing a mammalian animal population having an essentially identical metabolome comprising compiling a mammalian animal population being of essentially the same age, keeping said mammalian animal population for a time period sufficient for acclimatization under the following housing conditions: (i) constant temperature, (ii) constant humidity, (iii) physical separation of the mammalian animals of the mammalian animal population, (iv) feeding ad libitum, wherein the food to be fed is essentially free of chemical or microbial contaminants, (v) drinking liquid ad libitum, wherein the drinking liquid is essentially free of chemical or microbial contaminants, (vi) constant illumination period, and providing the mammalian animal population after said time period. Further, the present invention relates to methods for the identification of a compound which effects the metabolome of a mammalian animal, or methods for the identification of a marker for such compounds. Moreover, the present invention encompasses methods for the identification of such compounds or markers thereof comprising metabolically analyzing a sample from at least one mammalian animal of a mammalian animal population.

State-of-the-art techniques of phenotype analysis of organisms comprise, inter alia, analysis of the entire genome of said organism, called genomics, analysis of the entirety of the proteins, called proteomics, and the analysis of the entirety of RNA transcripts. More recently, these fundamental techniques of phenotypic analysis have been completed by the analysis of the metabolome, the entirety of metabolites, of an organism. This analysis is called metabolomics or, sometimes, metabonomics. Metabolomics can be defined as the qualitative and quantitative determination of all low molecular weight compounds (i.e. metabolites) in an organism's cell or body fluid at a specific time and under specific environmental conditions. An advantage of metabolomics is that effects caused by exogenous factors can be immediately monitored due to metabolic changes which usually appear much earlier than changes, if any, in the proteome or even in the genome.

Various techniques have been described already for the analysis of the metabolome of an organism. These techniques include, for instance, mass spectroscopy, NMR, Fourier transform infrared (FT-IR) spectroscopy, and flame ionisation detection (FID), optionally coupled to chromatographic separation techniques such as liquid chromatography, gas chromatography or HPLC. These techniques allow high-throughput screening of large populations of organisms for variations in the composition of their metabolome, i.e. they allow to determine a metabolic phenotype. An organism's metabolic phenotype is the entirety of its metabolites (metabolome) at a certain time and is the result of complex interactions of its genetic composition and the living environment of the organism. Accordingly, differences in the metabolome of the individuals of a population of organisms may be caused not only by differences in the genome but also by environmental factors which influence metabolic activities. Metabolomics, thus, allows to determine even effects of exogenous factors which do not influence the genome, transcription or proteome of an organism immediately. For instance, a toxic compound is harmful for an organism but must not necessarily cause changes in the genome of said organism. Currently, laboratory animals of a mammalian animal population are individually kept under equal and controlled environmental and nutritional conditions (US-A-5163380, US-A-346488).

At present, a drawback in metabolomics in comparison to other principle phenotypic analysis approvals is that organisms and, in particular, mammalian animals which are used in metabolic studies in the prior art do not share a common metabolome at the beginning of a study. In genomics, for instance, a population having an essentially identical genome can be easily provided by state-of-the-art cloning techniques. However, it would be highly desirable to detect metabolic influences of exogenous factors, such as toxic compounds or drugs under optimized conditions. Techniques for establishing mammalian animals having an essentially identical metabolome are the basis for a reliable and efficient metabolome analysis. Notwithstanding the need therefore, such techniques are not yet described.

Accordingly, the technical problem underlying the present invention must be seen as the provision of methods for complying with the aforementioned needs, i.e. providing a mammalian animal population which has an essentially identical metabolome suitable for reliable metabolome studies and reliable analysis. The technical problem is solved by the embodiments characterized in the claims and described herein below.

Accordingly, the present invention relates to a method for providing a mammalian animal population having an essentially identical metabolome comprising:
a) compiling a mammalian animal population being of essentially the same age;
b) keeping said mammalian animal population of step a) for a first time period sufficient for acclimatization under the following housing conditions:
   i) constant temperature;
   ii) constant humidity;
   iii) physical separation of the mammalian animals of the mammalian animal population;
   iv) feeding ad libitum, wherein the food to be fed is essentially free of chemical or microbial contaminants;
   v) drinking ad libitum, wherein the drinking liquid is essentially free of chemical or microbial contaminants;
   vi) constant illumination period; and
c) providing the mammalian animal population of step b) after said first time period.

The term "method for providing" as used herein does, preferably, not encompass methods of treatment of the mammalian animal body. Specifically, the method referred to herein is neither suitable for medical treatment or therapy of any disease or disorder nor suitable to improve or maintain the general well-being of the mammalian animals of the mammalian animal population in comparison to mammalian animals kept under other physiological conditions. Moreover, the term does not include any breeding techniques per se.

The term "mammalian animal population" relates to a plurality of mammalian animals. A plurality of mammalian animals as used herein is a group of mammalian animals consisting of at least two, preferably, 5 to 120, more preferably 5 to 25 of said mammalian animals per sex, dose and time point. The mammalian animals of the mammalian animal population are of the same species and, preferably, of the same strain. Preferred mammalian animals to be used in the method of the present invention are rodents, more preferably, rats or mice. If rats are used for the method of the present invention, it is furthermore preferred that these rats are wistar (CrlGlxBrlHan: Wi) rats (Charles River, USA). Further preferred rat strains are: BDIX strains; BDIX/CrCrl, BDlX/OrlCrl, Brown Norway strains; BN/CrlCrlj, BN/Crl, BN/OrlCrl, BN/OrlCrl, BN/SsNHsdMcwiCrl, Buffalo strain; BUF/CrCrl; Fischer strains, F344/DuCrl, F344/DuCrlCrlj, F344/lcoCrl, F344/DuCrl, SASCO Fischer strain, F344/NCrl; Copenhagen strains, COP/CrCrl, COP/NCrl; Cotton strain, COT/NCrl; Dahl/SS strain, SS/JrHsdMcwiCrl; Fawn Hooded strain, FHH/EurMcwiCrl; GK strain, GK/Crlj; Lewis strains, LEW/CrlCrl; LEW/Crl; Noble strain, NBL/CrlCrl; SHR strains, SHR/NCrlCrlj, SHR/NCrl, SHR/NCrl; WAG strain, WAG/RijCrl; Wistar Furth strains, WF/CrCrl, WF/lcoCrl; WKY strains, WKY/NCrl, WKY/NCrlCrlj, WKY/NlcoCrl; ZDF strains, ZDF/Crl-*Lepr^{fa}*, ZDF/Crl-*Lepr^{fa}*; CD strains, Crl:CD(SD), Crlj:CD(SD), Crl:CD(SD), Crl:CD(SD); SASCO SD strain, Crl:SD; OFA strains, Crl:OFA(SD), Crl:OFA(SD)-hr; DIO strain, Crl:CD(SD)DR; DR strain, Crl:CD(SD)DR; Donryu strain, Crlj:DON; LEC strain, Crlj:LEC; Wistar strains, Crlj:Wl, Crl:Wl; Wistar Han strains, Crl:Wl(Han), Crl:Wl(Han), Crl:Wl (Han), Crl:Wl(Han); Wistar WU strain, Crl:Wl(WU) or any strain which is derived from the aforementioned strains by way of cross breeding or genetic manipulation. The aforementioned strains are well known in the art and commercially available, e.g., via Charles River, USA or Harlan, USA. If mice are used for the method of the present invention, it is preferred that the mice are C57BL/GNCrl mice (Charles River, USA). Other preferred mouse strains are: CD-1 strains, Crlj:CD1(ICR), Crl:CD1(ICR), Crl:CD1(ICR), Crl:CD1(ICR), Crl:CD1(ICR); CF-1 strains, Crl:CF1, Crl:OF1; CFW strains, Crl:CFW(SW); NMRI strains, Crl:NMRI, Crl:NMRI (Han); PGP strain, Crl:CF1-*Abcb1a*, SKH1 strains, Crl:SKH1-*hr*, Crl:SKH1-*hr*; SKH2 strain, Crl:SKH2-*hr*; A/J strain, A/J; 129 strains, 129S2/SvPasCrl, 129S2/SvPasCrl; 129/S1/Sv-*p⁺Tyr⁺Kit^{sl-J}*/Crl, 129/s1/svlmJ; AKR strain, AKR/NCrl; BALB/c strains, BALB/cAnNCrlCrlj, BALB/CAnNCrl, BALB/cByJ; C3H strains, C3H/HeJCrl, C3H/HeNCrlCrlj, C3H/HeNcrl, C3H/HeOuJ; C57BU6 strains, C57BU6JCrl, C57BL/6J, C57BL/6JCrl. C57BL/6NCrlCrlj, C57BL/6NCrl, C57BL/6J, C57BU6JCrl, C57BL/6J; C57BU10 strains, C57BL/10JCrl, C57BL/10JCrl; CBA strains, CBA/CaCrl, CBA/J, CBA/JNCrlj, CBA/JNCrljCrlg; CB17 strain, CB17/lcrCrl; DBA/1 strains, DBA/1JCRL, DBA/1JNCrlj, DBA/1NlcoCrl; DBA/2 strains, DBA/2J, DBA/2NCrlCrlj, DBA/2NCrl, DBA/2JCrl; FVB strain, FVB/NCrl; NC strain, NC/NgaTndCrlj; NOD strain, NOD/LtJCrl; SJL strains, SJL/JOrlCrl, SJL/JOrl/CrlCrlj, SJUJ, SJL/JCrl; B6C3F1 strains, B6C3F1/Crl, B6C3F1/Crlj; B6CBAF1 strains, B6CBAF1/Crl, B6CBAF1/J, B6CBAF1/Crl; BDF1 (B6D2F1) strains, B6D2F1/Crl, B6D2F1/J, B6D2F1/Crlj; B6SJLF1 strain, B6SJLF1/J; C3D2F1 strain, C3D2F1/Crl; CDF1 (CD2F1) strains, CD2F1/Crl, CD2F1/Crlj, CD2F1/Crl; CBAB6F1 strain, CBAB6F1/Crl; CB6F1 strain, CB6F1/Crl; NMRCF1 strain, NMRCF1/Crl or any strain which is derived from the aforementioned strains by way of cross breeding or genetic manipulation. The aforementioned strains are well known in the art and commercially available, e.g., via Charles River, USA or Harlan, USA. Further preferred mammalian animals are dogs. Preferred dogs encompass beagles, more preferably, the beagle strains HsdRdg:DOBE or HsdHFr:DOBE or strains which are derived from the aforementioned strains by way of cross breeding or genetic manipulation. The aforementioned strains can be purchased from Harlan, USA. Other preferred beagles are from an inbreed strain and can be purchased from BASF AG, Germany. However, the mammalian animals to be used are not limited to the mammalian animals mentioned before and may further be selected from the group consisting of: cats, horses, cows, sheep, goats, rabbits.

The term "metabolome" as used herein refers to the entirety of metabolites in a cell, tissue, organ or entire mammalian animal. The metabolites are, preferably, small molecule compounds, such as substrates for enzymes of metabolic pathways, intermediates of such pathways or the products obtained by a metabolic pathway. Metabolic pathways are well known in the art and may vary between species. Preferably, said pathways include at least citric acid cycle, respiratory chain, photosynthesis, photorespiration, glycolysis, gluconeogenesis, hexose monophosphate pathway, oxidative pentose phosphate pathway, production and β-oxidation of fatty acids, urea cycle, amino acid biosynthesis pathways, protein degradation pathways such as proteasomal degradation, amino acid degrading pathways, biosynthesis or degradation of: lipids, polyketides (including e.g. flavonoids and isoflavonoids), isoprenoids (including eg. terpenes, sterols, steroids, carotenoids, xanthophylls), carbohydrates, phenylpropanoids and derivatives, alcaloids, benzenoids, indoles, indole-sulfur compounds, porphyrines, anthocyans, hormones, vitamins, cofactors such as prosthetic groups or electron carriers, lignin, glucosinolates, purines, pyrimidines, nucleosides, nucleotides and related molecules such as tRNAs, microRNAs (miRNA) or mRNAs. Accordingly, small molecule compound metabolites are preferably composed of the following classes of compounds: alcohols, alkanes, alkenes, alkines, aromatic compounds, ketones, aldehydes, carboxylic acids, esters, amines, imines, amides, cyanides, amino acids, peptides, thiols, thioesters, phosphate esters, sulfate esters, thioethers, sulfoxides, ethers, or combinations or derivatives of the aforementioned compounds. The small molecules among the metabolites may be primary metabolites which are required for normal all function, organ function or animal growth, development or health. Moreover, small molecule metabolites further comprise secondary metabolites having essential ecological function, e.g. metabolites which allow an organism to adapt to its environment. Furthermore, metabolites are not limited to said primary and secondary metabolites and further encompass artifical small molecule compounds. Said artificial small molecule compounds are derived from exogenously provided small molecules which are administered or taken up by an organism but are not primary or secondary metabolites as defined above. For instance, artificial small molecule compounds may be metabolic products obtained from drugs by metabolic pathways of the mammalian animal. Moreover, metabolites further include peptides, oligopeptides, polypeptides, oligonucleotides and polynucleotides, such as RNA or DNA. More preferably, a metabolite has a molecular weight of 50 Da (Dalton) to 30,000 Da, most preferably less than 30,000 Da, less than 20,000 Da, less than 15,000 Da, less than 10,000 Da, less than 8,000 Da, less than 7,000 Da, less than 6,000 Da, less than 5,000 Da, less than 4,000 Da, less than 3,000 Da, less than 2,000 Da, less than 1,000 Da, less than 500 Da, less than 300 Da, less than 200 Da, less than 100 Da. Preferably, a metabolite has, however, a molecular weight of at least 50 Da. Most preferably, a metabolite in accordance with the present invention has a molecular weight of 50 Da up to 1,500 Da.

The term "essentially identical metabolome" means that all individuals of the mammalian animal population provided by the method of the present invention have synchronized metabolic activities resulting (i) in the presence of essentially the same metabolites in the metabolome of each individual of the population and (ii) in amounts of said metabolites which are essentially identical for each of the individuals of the mammalian animal population. Preferably, synchronized metabolic activities as used herein means that all metabolic pathways which affect the metabolome of the mammalian animals are active in essentially the same cells, tissues or organs at essentially the same time, gene expression levels are in all mammalian animals are essentially identical and artificial small molecules are available for all mammalian animals in essentially identical amounts. It is to be understood that the metabolite amounts may vary between the individuals of the mammalian animal population within certain limits. Whether the same metabolites are present in essentially the same amounts in the individuals of the mammalian animal population referred to in accordance with the present invention, can be determined by various qualitative and/or quantitative compound analysis techniques. These techniques include but are not limited to chromatographic techniques for compound separation coupled to compound analysis techniques such as mass spectrometry (MS), Fourier transform ion resonance (FT-IR) spectroscopy, or FiD. Preferred in accordance with the present invention is a quantitative and/or qualitative determination of the metabolites by using liquid- and gas- chromatography coupled mass spectrometry (LC-MS and GC-MS). Details of said preferred methods are described below. Preferably, an mammalian animal population has an essentially identical metabolome if the mass spectra generated by one of these techniques are essentially identical for each individual of the population. Said mass spectra are essentially identical if all major peaks which are detectable by, e.g., a commercial peak annotation algorithm, such as ChemStation (Agilent Technologies, USA), Analyst (MDS SCIEX, Canada) or AMDIS (NIST, USA), appear in all spectra at essentially identical chromatographic retention times. As discussed above, minor variations are tolerable if they do not result in statistically significant differences. Whether a variation is minor in accordance with the present invention may be determined by suitable statistical algorithms well known to the person skilled in the art such as principal component analysis (PCA) or partial least square tests (PLS). Preferably, an essentially identical metabolome of the mammalian animals of the mammalian animal population can be determined together in a multivariate analysis (e.g., PCA) or hierachical clustering.

The term "compiling" as used herein refers to selecting the mammalian animals from any source to establish the mammalian animal population to be subjected to the method of the present invention. Accordingly, the mammalian animals may be progeny of the same mother mammalian animal or progeny of different mother mammalian animals. In case a single progeny of one mother mammalian animal is used as a source, either the entire progeny may be used for compiling the mammalian animal population or selected mammalian animals of the progeny may be used. Compiling as used herein is carried out with respect to the age of the mammalian animals, i.e. all individuals of the population shall have essentially the same age as described below in detail. However, further characteristics may be taken into account. In addition, such as weight, sex, overall appearance (e.g. only healthy animal by appearance may be selected).

The term "essentially the same age" means that the mammalian animals have a comparable status of development, e.g. the mammalian animals may be embryos, juveniles or adults. A preferred age of the mammalian animals to be used in the method of the present invention is an age of the adolescence stage, preferably young adolescence stage. The mammalian animals of the mammalian animal population, preferably, have an age with the range of X ± n day, wherein X is the envisaged age of the mammalian animal population and n is selected from an integer of 1 to 5 days, and, more preferably, n is 1 day. In other words, a given mammalian animal of the population shall be at most one day older or younger than the average age of the mammalian animals of the mammalian animal population. Most preferably, all mammalian animals of the population are of age X. Such mammalian animals can be provided by compiling mammalian animals which are progeny of one litter, i.e. littermates, or which are compiled from different litters from the same day. In case embryos are to be used, it is to be understood that essentially the same age relates to their developmental stages. The developmental stages of embryos from various species can be determined by techniques well known in the art. They may be calculated, e.g., based on the time point of fertilization. Moreover, individual embryos can be developmentally staged due to known morphological features. Moreover, in case embryos are used, it is further to be understood that the pregnant mothers carrying said embryos shall be kept under the conditions referred to herein.

If rats or mice are used as mammalian animals in the method of the present invention, it is preferred that the mammalian animals are of age X ± 1 day, wherein X is selected from an integer of 10 to 100 days, more preferably, an integer of 20 to 80 days, and, most preferably, X is 63, 64 or 65 days after birth. Most preferably, X is 64 days after birth. If dogs are used as mammalian animals in the method of the present invention, X is preferably 6 months after birth.

The term "keeping" as used in accordance with the method of the present invention refers to particular housing, feeding, drinking and environmental conditions which are applied to the mammalian animals of the mammalian animal population. It is preferred that the mammalian animals are kept under conditions as set forth in the OECD Guideline For The Testing Of Chemicals No: 407. Moreover, particular conditions are described as follows.
i) All mammalian animals of the mammalian animal population are kept under the same constant temperature. Care should be taken to choose a temperature for carrying out the method of the present invention which does not stress the mammalian animals. Preferably, temperature should be 20 to 24 ± 2°C, more preferably 22 ± 2°C, most preferably 22, 23 or 24 °C.
ii) Moreover, all mammalian animals of the mammalian animal population are kept under the same constant humidity. The humidity should be at least 30 %, but should not exceed 70 %. However, in rare exceptional situations (such as during room or cage clearing) humidity may even exceed 70 %.
   Preferably, humidity is 50-60 %.
iii) Physical separation of the mammalian animals of the mammalian animal population has been found to be also important for the method of the present invention. Accordingly, each mammalian animal of the mammalian animal population must be kept in a separate space, e.g. a separate cage.
iv) The mammalian animals of the mammalian animal population are fed ad libitum. The food to be used must be essentially free of chemical or microbial contaminants. The standards to be applied are laid down in Fed. Reg. Vol. 44, No. 91, May 09, 1979, p. 27354. Most preferably, microbial contaminants such as bacteria are below 5 x 10⁵ cells per g of food. Such food may be purchased from Provimi Kliba SA Kaiseraugst (Switzerland) as Ground Kliba mouse/rat maintenance diet "GLP" meal.
v) The mammalian animals of the mammalian animal population are supplied ad libitum with a drinking liquid. Preferably, said liquid is water. However, other liquids on water basis may be used as well. Such liquids may comprise, for instance, nutritions, vitamins or minerals which are required by the animals. If water is used as drinking liquid, the water shall be free of chemical and microbial contaminants as laid down in the European Drinking Water Directive 98/83/EG.
vi) Finally, each mammalian animal of the mammalian animal population must be subjected to the same constant illumination periods. Constant illumination is achieved, preferably, by artificial lightning (normal solar spectrum). The illumination period is 12 hours light followed by 12 hours darkness. Then the illumination period starts again. A preferred illumination period, thus, is 12 hours light, from 6:00 to 18:00, and 12 hours darkness, from 18:00 to 6:00.

The aforementioned housing conditions can be applied to the mammalian animals by using a common storage space for the cages comprising the physically separated mammalian animals. Said common storage space may be a mammalian animal room or house. By keeping all mammalian animals of the population in the same room, constant humidity, temperature and illumination period can be easily achieved by regulating these parameters for the entire room or house. Regulation of the parameters is preferably assisted by automation and the parameters are constantly monitored.

Under the term "first time period sufficient for acclimatization" it is to be understood that the mammalian animals of the mammalian animal population must be kept under the aforementioned particular housing conditions for a time period which allows synchronization of the metabolic activities of the mammalian animals so that the mammalian animals are acclimatized and have essentially the same metabolome. Specifically, the said first time period shall be of sufficient length as to allow all individuals of the population to adopt the same circadian rhythm, food digestion rhythm, or quiescence/movement periods. Moreover, the first time period shall allow each mammalian animal to adjust its biochemical and physiological parameters in response to the applied environmental conditions, such as humidity and temperature. Preferably, said first time period has a length of 5 to 10 days, more preferably 6 to 8, and most preferably 7 days.

In the studies underlying the present invention, it has been surprisingly found that a mammalian animal population having essentially the same metabolome can be provided by the method of the present invention. The superior results achieved by said method depend, however, on strictly obeying the aforementioned housing conditions and the criteria for compiling the mammalian animal population, i.e. compiling with respect to the age of the mammalian animals of the animal population. The later issue is particular surprising because according to the OECD Guideline No: 407, loc. cit., care should be taken with respect to the weight or sex when selecting or compiling mammalian animals for analytical purposes. Advantageously, by using the method of the present invention, it is possible to generate an mammalian animal population which can be applied for comparative metabolomics. Because of the essentially identical metabolome it is possible to reliably and efficiently study, e.g., toxic effects of compounds or to determine modes of action of compounds such as drugs or drug candidates. Moreover, the method of the present invention can be easily implemented in already existing mammalian animal facilities and is, thus, cost effective.

The present invention also encompasses a method for the identification of a compound which effects the metabolome of a mammalian animal comprising:
a) providing a mammalian animal population using the steps of the method of claim 1;
b) administering to said mammalian animal population a compound suspected to effect the metabolome of a mammalian animal; and
c) analyzing the metabolome of the mammalian animal population of step b);
d) carrying out necropsy of each of the mammalian animals of the mammalian animal population.

The term "identification" as used herein means that the method of the present invention is to be applied for identifying or screening compounds which effect the metabolome of a mammalian animal. Accordingly, it is envisaged that the method will provide data which allow to identify a compound which effects the metabolome of a mammalian animal. Such data may be obtained by various techniques for analyzing the metabolome of a mammalian animal described in detail below. Data may be in the form of raw data or processed data. Preferably, the result of the metabolome analysis in form of raw data or processed data will be compared with corresponding data obtained from a reference. Accordingly, it is to be understood that identification as used in accordance with the method of the present invention may require further steps. However, these further steps relate to well known techniques in comparative analysis including those mentioned before and below. Moreover, identification as used herein encompasses preferably identification of the compounds' property to elicit an effect to the metabolome of a mammalian animal, in principle, regardless of the kind of the effect or mode of action. Moreover, the term further encompasses, preferably, the identification of the kind of effect which is elicited or event the precise mode of action. Therefore, the method of the present invention may be also used for identifying a particular metabolic pathway influenced by a compound or a certain mode of action of a compound. More preferably, the method for identification described above and below may be used to

The term "compound which effects the metabolome of a mammalian animal" encompasses all classes of chemical compounds. Preferably, a compound as used herein is a small molecule compound, a peptide, a polypeptide or a polynucleotide. Small molecules as referred to in accordance with the present invention include inorganic and organic molecules having a low molecular weight, preferably a molecular weight which is lower than 30,000 kDa, more preferably lower than 20,000 Da, 10,000 Da, 8,000 Da, 5,000 Da, 3,000 Da, 2,000 Da, 1,000 Da or 500 Da. Most preferably, said compound is either suspected to be a toxic compound, a nutrient, a nutraceutical or a therapeutically active compound (i.e. a drug). A drug in accordance with the present invention will have a therapeutic effect on the mammalian animal, i.e. it will treat or ameliorate a medical condition. Said medical condition may be a disease or disorder or an impaired well-being of a mammalian animal. Amelioration or treatment can be monitored by the appearance or degree of the symptoms of the said disease, disorder or impairment. Drugs in accordance with the present invention also include drug precursors which are converted in vivo into the therapeutically active drug and compounds suspected to be drugs, i.e. drug candidates. Peptides or polypeptides to be used in accordance with the method of the present invention include naturally occurring peptides and polypeptides as well as artificial peptides and polypeptides. Naturally occurring peptides and polypeptides can be obtained from all kinds of organisms including plants, animals, fungi, bacteria or viruses. Artificial peptides or polypeptides can be generated by random peptide synthesis techniques, for instance. Preferred peptides or polypeptides to be used in accordance with the present invention are those which directly or indirectly influence metabolic activities or metabolic pathways in a mammalian animal according to the present invention. More preferably, said polypeptides or peptides have a therapeutic value, such as peptide hormones, growth factors, survival factors, cytokines, receptors for said polypeptides, antibodies or biologically active fragments thereof. Polynucleotides to be used in accordance with the method of the present invention are RNA or DNA molecules. Preferably, said polynucleotides encode peptides or polypeptides which directly or indirectly influence metabolic activities or metabolic pathways. For instance, suitable polynucleotides may encode enzymes, preferably those of metabolic pathways, peptides or polypeptides as specified above or peptides or polypeptides which regulate the expression of the aforementioned peptides or polypeptides, such as transcription factors. Moreover, polynucleotides to be used in the method of the present invention as compounds may be polynucleotides which interfere with gene expression (i.e. transcription or translation), such as anti-sense RNA molecules, anti-sense oligonucleotides or small interfering RNA (siRNA) to be used for RNA interference technology (RNAi).

The compounds referred to in accordance with the present invention are screened for their capability to effect the metabolome of a mammalian animal. Thus, a compound in the sense of the present invention will alter the composition of the metabolome of a mammalian animal upon administration. Said effect to the metabolome of a mammalian animal may be either a qualitative or quantitative effect. A qualitative effect to the metabolome as used herein means that at least one metabolite of the metabolome of the mammalian animal is absent or at least one additional metabolite is present after administration of the compound. A quantitative effect as used in accordance with the present invention means that the amount of at least one metabolite is altered, i.e. higher or lower, after administration of said compound. It is to be understood that a compound which effects the metabolome of a mammalian animal may also influence the biological function of the cells, the tissues or the organs of said mammalian animal and may cause intoxications, health improvements, health impairments or disorders.

The term "administering" as used herein includes all techniques by which the compounds may be provided systemically to the mammalian animal. Moreover, the term encompasses techniques for delivering the compounds to the suspected site of action such as a potential target tissue or organ, i.e. topical administration. The compounds to be administered in accordance with the present invention may be comprised in a composition further comprising suitable carriers, such as pharmaceutical carriers, excipients and/or diluents. Examples for well known diluents include phosphate-buffered saline solutions, water, emulsions, such as oil/water emulsions, various types of wetting agents, sterile solutions etc. Administration of the compounds or the aforementioned suitable compositions may be effected by different ways, e.g. by intravenous, intraperitoneal, subcutaneous, intramuscular, topical, intradermal, intranasal or intrabronchial administration. Preferably, administration is achieved by oral administration, most preferably the compound is admixed to the drinking liquid or the food. Suitable treatment and dosage regimens are well known to the person skilled in the art and a preferred treatment and dosage regimen is described in the Examples. Preferably, the mammalian animals of the test mammalian animal population (i.e. the mammalian animal population to which the compound is to be administered) are subdivided into at least one group of males and at least one group of females and at least one high and at least one low dose group.

The term "analyzing the metabolome" as used in accordance with the present invention refers to techniques for quantitatively or qualitatively analyzing the metabolome. In a first step, said qualitatively or quantitatively analyzing the metabolome comprises determining qualitatively and qualitatively the composition of the metabolome, i.e. the metabolites. Means and methods for qualitatively analyzing the metabolome comprise those which are capable to determine the presence or absence of the metabolites comprised by the metabolome. Means and methods for quantitatively analyzing the metabolome are those which determine the amount of the said metabolites. It is to be understood that there are means and methods which may determine the presence or absence as well as the amount of the metabolites and, thus, allow a combination of qualitative and quantitative analysis. Moreover, in accordance with the methods of the present invention it is not necessarily required to determine the entire metabolites of the metabolome. Rather, analysis of the metabolome may be carried out by determining the presence or absence or the amount of a portion of the metabolites found to be characteristic, a predetermined set of metabolites or a metabolic profile for the metabolome. Characteristic or predetermined metabolites comprise known metabolites as well as so called known unknowns. The later ones are metabolites which are merely known from their signal in, e.g., a mass spectra. The chemical nature of said known unknowns, however, is not precisely known. A metabolic profile as used herein relates to every kind of unique identifier for a certain metabolome, i.e. a fingerprint of a metabolome. Qualitative or quantitative analysis of the metabolome is, preferably, carried out by using compound analysis techniques. Suitable devices for such determination of compounds are well known in the art. Preferably, mass spectrometry is used in particular gas chromatography mass spectrometry (GC-MS), liquid chromatography mass spectrometry (LC-MS), direct infusion mass spectrometry or Fourier transform ion-cyclotrone-resonance mass spectrometry (FT-ICR-MS), capillary electrophoresis mass spectrometry (CE-MS), high-performance liquid chromatography coupled mass spectrometry (HPLC-MS), quadrupole mass spectrometry, any sequentially coupled mass spectrometry, such as MS-MS or MS-MS-MS, inductively coupled plasma mass spectrometry (ICP-MS), pyrolysis mass spectrometry (Py-MS), ion mobility mass spectrometry or time of flight mass spectrometry (TOF). Most preferably, LC-MS and/or GC-MS are used as described in detail below. Said techniques are disclosed in, e.g., Nissen, Journal of Chromatography A, 703, 1995: 37-57, US 4,540,884 or US 5,397,894. As an alternative or in addition to mass spectrometry techniques the following techniques may be used for compound determination: nuclear magnetic resonance (NMR), magnetic resonance imaging (MRI), Fourier transform infrared analysis (FT-IR), ultra violet (UV) spectroscopy, refraction index (RI), fluorescent detection, radiochemical detection, electrochemical detection, light scattering (LS), dispersive Raman spectroscopy or flame ionisation detection (FID). These techniques are well known to the person skilled in the art and can be applied without further ado.

In a further step, analyzing the metabolome as used herein, preferably, includes a comparison of the metabolites, amounts of metabolites or the metabolic profile to a corresponding reference. Said reference may be derived from a metabolome analysis of a mammalian animal population to which no compounds suspected to effect the metabolome of a mammalian animal has been administered, whereby it is preferred that this reference mammalian animal population has been treated in an otherwise identical manner. Further suitable references for a comparison in accordance with the present invention may be derived from a metabolome analysis of mammalian animals to which compounds have been administered which are known to effect the metabolome. Such compounds may be compounds which are toxic or which are therapeutically active via a known mode of action. Thus, by using mammalian animals treated with the aforementioned compounds known to effect the metabolome as reference, it may not only be determined whether a new compound has an effect to the metabolome in general. Rather, it may be further determined whether said new compound elicits toxic effects or may be therapeutically active via a certain mode of action or has at least the potential to do so. Preferably, the resulting data of a metabolome analysis of the references described before are stored and provided in form of a suitable database. Moreover, data of a metabolome analysis carried out by the method of the present invention for new compounds may also serve as reference for other new compounds and may be stored and provided also by the said data base for further analysis. Comparing as used herein encompasses comparison of the raw data obtained by the analysis of the metabolome or any kind of processed data derived from said raw data. Suitable means and methods for data comparison are well known in the art and include, for instance, principal component analysis (PCA) or partially least square tests (PLS). In principle, any statistical test which allows to determine whether metabolites, amounts thereof or a metabolic profile as described above differs significantly between different mammalian animals or time points of determination is suitable for carrying out the comparison referred to herein. The aforementioned differences can, in principle, be determined by pattern recognition algorithms, statistical test algorithms and/or multivariate algorithms, e.g., Principal Component Analysis (PCA), Simple Component Analysis (SCA), Independent Component Analysis (ICA), Principal Component Regression (PCR), Partial Least Squares (PLS), PLS Discriminant Analysis (PLS-DA), Support Vector Machines (SVM), Neural Networks, Bayesian Networks, Bayesian Learning Networks, Mutual Information, Backpropagation Networks, symmetrical Feed-Forward Networks, Self-Organizing Maps (SOMs), Genetic Algorithms, Hierarchical or K-Mean Clustering, Anova, Student's t-Test, Kruskal-Wallis Test, Mann-Whitney Test, Tukey-Kramer Test or Hsu's Best Test. Data processing as referred to above may preferably include a data validation step. In said data validation step, inconsistent data are excluded from further analysis. Inconsistent data may result technical problems during the qualitative or quantitative determination of the composition of the metabolome. Therefore, it is envisaged that the technical parameters of the devices used for determination are constantly monitored and provided for data validation in a suitable database. Further validation of the data may be achieved by internal validation tools which statistically evaluate each measured value or parameter of a data set. For example, if mass spectrometry is used for analyzing the metabolome, a raw data set comprising peaks is generated. Said peaks appear at a certain position in a three-dimensional space consisting of a retention time range, an intensity range and a mass-to-charge (m/z) ratio range. Each of said peaks may be evaluated and confirmed by peak validation algorithms such as ChemStation (Agilent Technologies, USA), Analyst (MDS SCIEX, Canada) or AMDIS (NIST, USA).

Furthermore, mammalian animal- or housing-related data may be taken into account for data validation. For example, if the housing conditions vary, the metabolome of a mammalian animal of the mammalian animal population may be adversely influenced. Such an adversely influenced metabolome of a mammalian animal may, however, cause false positive or negative results. Therefore, it is preferred to also monitor mammalian animal- or housing-related data as described below in detail and to regard these data during data validation.

Moreover, data processing, preferably, includes normalization of the raw data with respect to internal references. For example, peaks which can be allocated to known metabolites may be used to normalize peaks for unknown metabolites within a data set obtained for a metabolome of an individual of the mammalian animal population with respect to, e.g., signal intensity.

Moreover, data processing may be further required to create coherent data by converting data in a numeric format, converting the data into a common unit format and/or dimensionally reducing the data. The data may be further integrated in that information relating to the sample or mammalian animal is allocated thereto. Suitable means and methods for creating such coherent data are disclosed in WO 03/046798.

The data processing steps and the comparison referred to above are preferably assisted by automation, e.g., by a suitable computer program which runs on a computer device. The computer device is, preferably, operatively linked to the various data bases referred to in accordance with the present invention. Accordingly, in one embodiment, the present invention also relates to a database or system of databases which comprise the results obtained by the method of the present invention. More preferably, such a database or system of databases comprises information relating to the metabolomes elicited by compounds which are suspect to affect the metabolome of an organism, such as toxic or therapeutically active compounds (i.e. drugs). Thus, the database may be used as a tool (reference database) for screening assays which aim to identify new drugs or toxic compounds by their mode of action reflected by the metabolome.

It is to be understood that the analysis of the metabolome shall be carried out after a time period sufficient to allow the compound to enter its effector cells, tissues or organs, i.e. an incubation time period. In other words, the analysis should be carried out after the compound has become bioavailable. Depending on the chemical nature of the compound, the person skilled in the art will know what time period shall be sufficient for bioavailability of said compound. Such a time period could be also defined in a pilot experiment. For example, a compound being of similar or identical chemical nature may be linked to a detectable label. Said labelled compound may then be administered to a mammalian animal. The time until the label becomes detectable in the suspected effector cells, tissues or organs will be determined and will serve as incubation time period for the method of the present invention.

The analysis of the metabolome also, preferably, comprises taking a sample from each mammalian animal of the mammalian animal population which will be further analyzed as described before. Suitable samples include cells, tissues or organs of the animal or body fluids including blood, plasma, serum, urine, spinal liquor. It is well known in the art, how such samples may be taken. Suitable techniques include blood sampling, biopsy, liquor sampling, cell sorting etc. A preferred schedule for sampling is described in the Examples. The sample may be subjected to pretreatments. Such pretreatments include enzymatic digestion of biological material which is not suitable for metabolome analysis, extraction procedures to obtain certain metabolites form the sample, fractioning of the sample, e.g., in order to obtain polar and/or non-polar fractions of metabolites, or derivatisation of the metabolites, e.g., prior to chromatography. Said pretreatment techniques are well known in the art and can be applied by the person skilled in the art without further ado.

Advantageously, due to providing the mammalian animal population in accordance with the present invention reliable comparative analysis of the metabolome can be carried out. The results of said reliable analysis in accordance with the method of the present invention will fasten drug discovery and toxicological assessments. Preferably, databases are created which comprise metabolic data (i.e. data on the qualitative and quantitative composition of metabolomes or metabolic profiles) for a mode of action of a compound or metabolic data which reflect administration of toxic compounds or drugs. The metabolic data of said data bases can be used as references when using the method of the present invention for a screen of further compounds suspected to effect the metabolome. It is envisaged that each screen will yield a more comprehensive view on modes of action. Moreover, by comparison with the said metabolic data (reference data) toxic compounds or therapeutically active compounds can be rapidly identified. Preferably, the method of the present invention is carried out in the high-throughput format.

In a preferred embodiment of the method of the present invention, the mammalian animal population is kept for a second time period after administering to the mammalian animal population the compounds suspected to effect the metabolome in step b) under the housing conditions described referred to above.

More preferably, analyzing of step c) is carried out at least once during said second time period or at least three times during the second time period, wherein the second and any further analysis is carried out after a period of time which is twice the period of time which passed since the previous analysis. Most preferably, the first analysis is carried out seven days after administration of the compound in step b).

It has been found in the study underlying the present invention that it is advantageous to evaluate the metabolome over a time range from acute up to long-term effects including intermediate time points. Thus, the method of the present invention allows repeated measurements during an investigation. Accordingly, analysing of the mammalian animals is preferably carried out at least once, preferably two times or most preferably at least three times during the second time period. The time points for analysis are to be selected as to include the early acute effects as well as the chronic or long-term effects. Suitable time points for the analysis can be determined by the person skilled in the art depending on the mammalian animal which is analyzed without further ado. Specifically, if a rodent mammalian animal population is used, such as a rat or mouse population, a time course of analysis is preferred in which the second and any further analysis is carried out after a period of time which is twice the period of time which passed since the previous analysis, i.e. if the first analysis is done 7 days after administration of the compound, the second analysis shall be done after 14 days and the third analysis shall be done after 28 days after administration. Preferably, if analysis is done by analyzing a body fluid sample, such as blood, it is preferred that the mammalian animals of the mammalian animal population will be kept for a fasting period (i.e. withdrawal of food) of about 16 to 20 hours before blood sampling. Moreover, carrying out the analysis during a constant time window allows to compensate for metabolic changes which may be caused for instance by the circadian rhythm of the mammalian animals of the mammalian animal population. Therefore, it is preferred that blood sampling and analysis is done within and at a constant time window for all analyses. Preferably analysis including sampling is done between 7.30 and 10.30 a.m. Moreover, the methods of the present invention may further encompass in addition to the analysis of the blood metabolome further investigations of the metabolome of different organs or body fluids of the mammalian animals of the mammalian animal population.

Moreover, in addition to the metabolome investigations, the method may comprise further investigations which do not relate to the metabolome. Specifically, it is envisaged that each mammalian animal of the mammalian animal population will after the metabolic analysis is completed, be pathologically investigated. Said investigation includes necropsy of the animals including careful examination of the external surface of the body, all orifices, the cranial, thoracic and abdominal cavities and their contents. Moreover, a pathologic analysis of the liver, the kidneys, the adrenals, the testis, the epididymidis, the thymus, the spleen, the brain and the heart shall be carried out as appropriate and as described in Guideline No. 407, loc. cit., for instance. Moreover, the histopathological examination shall be carried out, preferably, as described in the following:
The following tissues should be preserved in the most appropriate fixation medium for both the type of tissue and the intended subsequent histopathological examination: all gross lesions, brain (representative regions including cerebrum, cerebellum and pons), spinal cord, stomach, small and large intestines (including Peyer's patches), liver, kidneys, adrenals, spleen, heart, thymus, thyroid. trachea and lungs (preserved by inflation with fixative and then immersion), gonads, accessory sex organs (e.g. uterus, prostate), urinary bladder, lymph nodes (preferably one lymph node covering the route of administration and another one distant from the route of administration to cover systemic effects), peripheral nerve (sciatic or tibial) preferably in close proximity to the muscle, and a section of bone marrow (or, alternatively, a fresh mounted bone marrow aspirate). The clinical and other findings may suggest the need to examine additional tissues. Also any organs considered likely to be target organs based on the known properties of the test compound should be preserved.

Moreover, it also preferred that haematological and biochemical parameters of the mammalian animals of the mammalian animal population will be determined. It is preferred that data relating to the pathology and the haematological as well as biochemical parameters will be stored in a suitable database for each of the mammalian animals of the mammalian animal population as mammalian animal-related data. The stored data are preferably used for the evaluation of the metabolic data generated by the analysis of the metabolome and/or for data processing. In a preferred embodiment of the method of the present invention, taking into account this mammalian animal-related data allows to avoid false positive or false negative results because mammalian animals which shall in comparison to their parallels in a mammalian animal population an abnormal pathology, haematology or abnormal biochemical parameters may be excluded from data evaluation during data processing. Moreover, it is further preferred to include mammalian animal-related data on body weight, food consumption, drinking liquid consumption and clinical signs as well as possible abnormalities which appear when the mammalian animals are kept for the first and second time period as specified above.

Thus, in another preferred embodiment of the method of the present invention, said method further comprising monitoring body weight, food consumption, drinking liquid consumption and clinical signs of the mammalian animal population. Said monitoring may be, more preferably, be assisted by automation and the monitored data on body weight, food consumption, drinking liquid consumption and clinical science are collected in a database for each of the mammalian animal of the mammalian animal population.

Moreover, in light of the foregoing, the method of the present invention is, preferably, comprising monitoring abnormalities for each mammalian animal of the mammalian animal population. The term "abnormalities" as used herein refers to abnormalities which can be easily detected by maintenance stuff, i.e. abnormalities which do usually not require monitoring by a physician. More preferably, the abnormalities are automatically monitored and the data obtained from said monitoring are stored in a database for each individual of the mammalian animal population.

As referred to already above, in a preferred embodiment of the method of the present invention, said analyzing comprises comparing the metabolome of the mammalian animal population with a reference.

More preferably, comparing comprises generating a metabolic profile of the metabolome of the mammalian animals of the mammalian animal population and comparing said profile with a reference. Most preferably, a difference in the metabolic profiles is indicative for a compound which effects the metabolome of a mammalian animal. It is to be understood that the reference in this most preferred embodiment is a metabolic profile derived from an untreated mammalian animal.

The term "metabolic profile" means that a specific fingerprint is established during analysis for each metabolome of an mammalian animal of the mammalian animal population. Said metabolic profile may be derived from at least one signal (e.g. a peak in mass spectra) obtained from a mammalian animal or a sample thereof by the method of the present invention. More preferably, a metabolic profile is derived from a plurality of such signals. The signals may be obtained from a single metabolite or from a plurality of metabolites. It is to be understood that the primary signals may be further processed by suitable techniques as described above. Preferably, a three-dimensional data set is generated by using at least one time resolved separation technique and at least one mass resolved separation technique. Such a data set could be obtained, e.g., from chromatography coupled mass spectrometry as described above. Such a three-dimensional data set may be analyzed by conventional peak determining algorithms, such as ChemStation or AMDIS, which allow for specific detection of maxima and/or minima in said three-dimensional data set. The maxima and minima thus extracted will be the specific metabolic profile for a certain mammalian animal metabolome of a mammalian animal population. Moreover, the extracted signals, such as the maxima or minima of the peaks may be further processed into characteristic values as a function of the respective time and mass. It is to be understood that the metabolic profiles generated by the aforementioned techniques consist, preferably, of data sets which are dimensionally reduced and, thus, can be easily compared to each other by statistical tests, including principal component analysis (PCA) or partial least square tests (PLS). A difference in the metabolic profile of a metabolome of a test mammalian animal (i.e. a mammalian animal which has obtained a compound suspected to effect the metabolome of a mammalian animal) and a reference is an indicator that the compound indeed induces metabolic changes in the test mammalian animal. However as described already above, this does not necessarily mean that the quantitative composition of the metabolome (i.e. the number of compounds which are present) will be changed. Changes in the metabolic profiles may be also an indicator for an altered quantitative composition of the metabolome (i.e. the amount of the compounds which are present is altered). More preferably, the metabolic profiles may be compared to metabolic profiles comprised by a data base with reference profiles for known drugs, prodrugs or toxic compounds or their modes of action. Advantageously, the method of the present invention, thus, allows to make screening procedures for drugs and drug candidates more time-efficient since toxic or harmful effects of the compounds may be determined at an early stage of drug development. Further, toxicity tests can be easily carried out in a time-efficient manner since the onset of toxic events can be monitored by changes of the metabolic profiles. In particular, this is advantageous for compounds which elicit long-term toxic effects instead of immediate effects.

It is to be understood that differences or changes in the metabolome of a mammalian animal can be also analyzed by comparing specific metabolites of known or unknown chemical nature as opposed to a metabolic profile.

Therefore, in another preferred embodiment of the method of the present invention, comparing comprises comparing of at least one metabolite of the metabolome of the mammalian animal population with a reference. Most preferably, a difference in said at least one metabolite is indicative for a compound which effects the metabolome of a mammalian animal. It is to be understood that the reference in this most preferred embodiment is a metabolic profile derived from an untreated mammalian animal.

In another preferred embodiment of the method of the present invention and in light of the foregoing, said compound is a compound suspected to be toxic or a compound suspected to be a drug.

The present invention further relates to a method for the identification of a marker for a compound which effects the metabolome of a mammalian animal, comprising the steps of the method of the present invention as described before and the further step of providing a marker for said compound based on the analysis of the metabolome. In a preferred embodiment of the method of the present invention, said marker is a metabolic profile of the mammalian animal population which is altered compared to a reference. Most preferably, said marker indicates toxicity of a compound, a mode of action of a compound or a therapeutic activity of a compound.

Moreover, the present invention encompasses a method for identifying a compound which effects the metabolome of a mammalian animal comprising metabolically analyzing a sample from at least one mammalian animal of a mammalian animal population to which a compound suspected to effect the metabolome has been administered and wherein said mammalian animal population has been kept prior and after administration of the compound under the housing conditions referred to before.

The term "metabolically analyzing" as used herein, preferably, encompasses all means, methods and embodiments described before for analyzing the metabolome of a mammalian animal.

The term "sample" as used herein encompasses samples of biological material obtained from the mammalian animal to be investigated. Suitable sources for samples have been described above already.

In a preferred embodiment of the method of the present invention, said analyzing comprises comparing the metabolome from the sample of a mammalian animal of said animal population with a reference.

More preferably, comparing comprises generating a metabolic profile for the sample of a mammalian animal of the mammalian animal population and comparing said profile with a reference. Most preferably, a difference in the metabolic profile is indicative for a compound which effects the metabolome of a mammalian animal. It is to be understood that the reference in this preferred embodiment is a metabolic profile derived from an untreated mammalian animal.

Also more preferably, comparing comprises comparing at least one metabolite of the metabolome from the sample of a mammalian animal of the mammalian animal population with a reference. Most preferably, a difference in the said at least one metabolite is indicative for a compound which effects the metabolome of a mammalian animal. It is to be understood that the reference in this most preferred embodiment is a metabolic profile derived from an untreated mammalian animal.

Also encompassed by the present invention is a method for the identification of a marker for a compound which effects the metabolome of a mammalian animal comprising the steps of the aforementioned methods (based on samples) and the further step of providing said marker based on the analysis of the metabolome.

In a preferred embodiment of said method of the present invention, said marker is a metabolic profile or is at least one metabolite from the metabolome of the sample of a mammalian animal of the mammalian animal population which is altered compared to a reference. Most preferably, said marker indicates toxicity of a compound, a mode of action of a compound or a therapeutic activity of a compound.

The invention will be now illustrated by the following Examples. However, the Examples are merely for purpose of illustration and not meant to limit the scope of the invention.

### Example 1: Animal keeping

Rats of the strain CrlGlxBrlHan:Wi were purchased from Charles River, Sulzfeld, Germany having an age of 63 to 65 days. Each animal has been labelled by an ear tattoo, consecutively. Animals were kept under the following housing conditions:

| | |
|---|---|
| Air conditions: | Temperature 20-24 °C, humidity 30-70 %. Any deviations have been documented. |
| | |
| Illumination period: | 12 hours light from 6.00 to 18.00 hours, 12 hours darkness from 18.00 to 6.00 hours |
| | |
| Type of cage: | Wire cages, type DK III, BECKER & Co., Castrop-Rauxel, Germany |
| | |
| No. of animals per cage: | 1 |
| Type of diet: | Ground Kliba mouse/rat maintenance diet "GLP", meal, supplied by Provimi Kliba SA, Kaiseraugst (Switzerland), ad libitum |
| | |
| Watering: | Drinking water ad libitum |
| | |
| Acclimatization: | During the 7 day acclimatization period, the animals have been accustomed to the environmental conditions of the study and to the diet. |

### Example 2: Metabolic investigation of test compounds

Male and female wistar rats have been randomized and allocated to the dose groups before the beginning of the administration period on the basis of their weights. The animals have been treated with five different test compounds at a high and low dose level according to the following schedule shown in Table 1.

**Table 1:**

| Dose group | Test substance | Dose level (ppm in the diet) | No. of animals per sex | Animal no. | |
|---|---|---|---|---|---|
| | | | | males | Females |
| 00 | 0 | 0 | 10 | 1-10 | 61-70 |
| 01 | A | Low dose | 5 | 11-15 | 71-75 |
| 02 | A | High dose | 5 | 16-20 | 76-80 |
| 03 | B | Low dose | 5 | 21-25 | 81-85 |
| 04 | B | High dose | 5 | 26-30 | 86-90 |
| 05 | C | Low dose | 5 | 31-35 | 91-95 |
| 06 | C | High dose | 5 | 36-40 | 96-100 |
| 07 | D | Low dose | 5 | 41-45 | 101-105 |
| 08 | D | High dose | 5 | 46-50 | 106-110 |
| 09 | E | Low dose | 5 | 51-55 | 11-115 |
| 10 | E | High dose | 5 | 56-60 | 116-120 |

Blood sampling was carried out as indicated in the following time schedule shown in Table 2.

**Table 2:**

| Date | Phase of study/Examination | Date of study |
|---|---|---|
| | Experimental starting date: Arrival of the animals and start of acclimatization period | -6 |
| | Randomization of the animals | |
| | Start of administration period | 0 |
| | Blood sampling¹⁾²⁾ | 7 |
| | Blood sampling¹⁾²⁾ | 14 |
| | Blood sampling¹⁾²⁾ and necropsy | 28 |
| | Blood sample preparation | |
| | Evaluation of the clinical findings | |
| | Summary of the clinical results | |

| | | |
|---|---|---|
| 1) = Before necropsy/blood examination fasting period (withdrawal of food) of about 16 to 20 hours 2) = Between 07:30 and 10:30 | | |

During the experiment, a check for moribund and dead animals has been made twice, daily from Monday to Friday and once daily on Saturday, Sunday and public holidays. The animals will be checked daily for any clinical abnormal signs. Abnormalities and changes will be documented for each animal. The food consumption has been determined on study days 6, 13, 20 and 27. Drinking water consumption has been checked daily within the general observations. Body weight has been determined before the start of the administration period, in order to randomize the animals. During the administration period the body weight has been determined on study days 0, 6, 13, 20 and 27. The mean daily intake of the test substances have been calculated based upon individual values for body weight and food consumption. Means and standard deviations have been calculated using Dunnet's test.

Blood sampling was carried out as follows:
Before necropsy or blood sampling, food was withdrawn for about 16 to 20 hours (fasting period). Blood sampling was done between 7:30 and 10:30 a.m. Blood was taken from the retroorbital venous plexus of isoflurane anaesthesized animals. From each animal 1 ml of blood will be collected in plastic tubes with EDTA as anticoagulant (10µl of a 10% solution). The blood samples are centrifuged. The plasma of each sample is separated and transferred to another plastic tube. The precipitated erythrocytes are washed three times with 0.9% NaCl, and filled up ad 1 ml with sterile distilled water (Ampuwa®, Fresenius, Bad Homburg, Germany) in order to hemolyse red blood cells. Hemoglobin is determined in the hemolysed blood samples (40 µl hemolysate plus 160 µl 1.5% NaCl) with an automatic analyzer (ADVIA 120, Bayer AG, Fernwald, Germany). Blood, plasma and hemolysate are sampled and stored in original Eppendorf tubes. Transport and preparation of the samples are done under cooling with ice. At the end of sample preparation all samples are overlaid with an atmosphere of pure nitrogen, sealed with "Parafilm M" and stored at -80°C (under nitrogen atmosphere) until further prosecution of the samples (e.g., shipment).

After completion of the experiment, clinical pathology for each animal was determined. To this end all animals which survived the study have been sacrificed by decaptation under isoflorane anaesthesia (if final blood sampling was envisaged) or by CO₂ anaesthesia.

## Claims

1. A method for providing a mammalian animal population having an essentially identical metabolome comprising:
a) compiling a mammalian animal population being of essentially the same age;
b) keeping said mammalian animal population of step a) for a first time period sufficient for acclimatization under the following housing conditions:
i) constant temperature;
ii) constant humidity;
iii) physical separation of the mammalian animals of the mammalian animal population;
iv) feeding ad libitum, wherein the food to be fed is essentially free of chemical or microbial contaminants;
v) drinking ad libitum, wherein the drinking liquid is essentially free of chemical or microbial contaminants;
vi) constant illumination period; and
c) providing the mammalian animal population of step b) after said first time period.

2. The method of claim 1, wherein said mammalian animal population is a rodent population.

3. The method of claim 2, wherein said rodent is a rat or mouse.

4. The method of claim 3, wherein said age is within the range of X ± 1 day, wherein X is the envisaged age of the population.

5. A method for the identification of a compound which effects the metabolome of a mammalian animal comprising:
a) providing a mammalian animal population using the steps of the method of claim 1;
b) administering to said mammalian animal population a compound suspected to effect the metabolome of a mammalian animal;
c) analyzing the metabolome of the mammalian animal population of step b); and
d) carrying out necropsy of each of the mammalian animals of the mammalian animal population.

6. The method of claim 5, wherein the mammalian animal population is kept for a second time period after administering to the mammalian animal population a compound suspected to be toxic in step b) under the housing conditions described in claim 1.

7. The method of claim 6, wherein analyzing of step c) is carried out at least once during the said second time period.

8. The method of claim 6, wherein analyzing of step c) is carried out at least three times during the said second time period, wherein the second and any further analysis is carried out after a period of time which is twice the period of time which passed since the previous analysis.

9. The method of claim 8, wherein the first analysis is carried out seven days after administration of the compound in step b).

10. The method of any one of claims 5 to 9, further comprising monitoring body weight, food consumption, drinking liquid consumption and clinical signs of the mammalian animal population.

11. The method of any one of claims 5 to 10, further comprising monitoring abnormalities for each mammalian animal of the mammalian animal population.

12. The method of any one of claims 5 to 11, wherein analyzing comprises comparing the metabolome of the mammalian animal population with a reference.

13. The method of claim 12, wherein comparing comprises generating metabolic profile for the metabolome of a mammalian animal of the mammalian animal population and comparing said profile with a reference.

14. The method of claim 13, wherein a difference in the metabolic profiles is indicative for a compound which effects the metabolome of a mammalian animal.

15. The method of claim 12, wherein comparing comprises comparing of at least one metabolite of the metabolome of a mammalian animal of the mammalian animal population with a reference.

16. The method of claim 15, wherein a difference in the said at least one metabolite is indicative for a compound which effects the metabolome of a mammal.

17. The method of any one of claims 5 to 16, wherein said compound is a compound suspected to be toxic or a compound suspected to be a drug.

18. A method for the identification of a marker for a compound which effects the metabolome of a mammal comprising the steps of the method of any one claims 5 to 17 and the further step of providing a marker for said compound based on the analysis of the metabolome.

19. The method of claim 18, wherein said marker is a metabolic profile or is at least one metabolite of a mammal of the mammalian animal population which is altered compared to a reference.

20. The method of claim 18 or 19, wherein said marker indicates toxicity of a compound, a mode of action of a compound or a therapeutic activity of a compound.

21. A method for identifying a compound which effects the metabolome of a mammal comprising metabolically analyzing a sample from at least one mammal of a mammalian animal population to which a compound suspected to effect the metabolome of a mammal has been administered and wherein said mammalian animal population has been kept prior and after administration of the compound under the housing conditions described in claim 1.

22. The method of claim 21, wherein said analyzing comprises comparing the metabolome from the sample of a mammal of said mammalian animal population with a reference.

23. The method of claim 22, wherein comparing comprises generating a metabolic profile for the sample of an animal of the mammalian animal population and comparing said profile with a reference.

24. The method of claim 23, wherein a difference in the metabolic profile is indicative for a compound which effects the metabolome of a mammal.

25. The method of claim 22, wherein comparing comprises comparing at least one metabolite of the metabolome from the sample of a mammalian animal population with a reference.

26. The method of claim 25, wherein a difference in the said at least one metabolite is indicative for a compound which effects the metabolome of a mammal.

27. The method of any one of claims 21 to 26, wherein said compound is a toxic compound or drug.

28. A method for the identification of a marker for a compound which effects the metbolome of a mammal comprising the steps of the method of any one of claims 21 to 26 and the further step of providing said marker based on the analysis of the metabolome.

29. The method of claim 28, wherein said marker is a metabolic profile or is at least one metabolite from the metabolome of the sample of a mammal of the mammalian animal population which is altered compared to a reference.

30. The method of claim 28 or 29, when said marker indicating toxicity of a compound, a mode of action of a compound or a therapeutic activity of a compound.

## Patentansprüche

1. Verfahren zum Bereitstellen einer Säugetierpopulation mit im wesentlichen identischem Metabolom, wobei das Verfahren folgendes umfaßt:
a) Zusammenstellen einer Säugetierpopulation mit im wesentlichen demselben Alter;
b) Aufstallung der Säugetierpopulation von Schritt a) über einen ersten, zum Akklimatisieren ausreichenden Zeitraum unter den folgenden Bedingungen:
i) konstante Temperatur;
ii) konstante Feuchtigkeit;
iii) physische Trennung der Säugetiere von der Säugetierpopulation;
iv) ad-libitum-Fütterung, wobei das zu verabreichende Futter im wesentlichen frei von chemischen oder mikrobiellen Kontaminanten ist;
v) ad-libitum-Tränke, wobei die Trinkflüssigkeit im wesentlichen frei von chemischen oder mikrobiellen Kontaminanten ist;
vi) Dauerlichtperiode; und
c) Bereitstellung der Säugetierpopulation von Schritt b) nach diesem ersten Zeitraum.

2. Verfahren nach Anspruch 1, wobei es sich bei der Säugetierpopulation um eine Nagetierpopulation handelt.

3. Verfahren nach Anspruch 2, wobei es sich bei dem Nagetier um eine Ratte oder Maus handelt.

4. Verfahren nach Anspruch 3, wobei das Alter im Bereich von X ± 1 Tag liegt, wobei X das Zielalter der Population ist.

5. Verfahren zum Identifizieren einer Verbindung, die das Metabolom eines Säugetiers beeinflußt, wobei das Verfahren folgendes umfaßt:
a) Bereitstellen einer Säugetierpopulation unter Verwendung der Schritte des Verfahrens von Anspruch 1;
b) Verabreichen einer Verbindung, von der vermutet wird, daß sie das Metabolom eines Säugetiers beeinflußt, an die Säugetierpopulation; ,
c) Analysieren des Metaboloms der Säugetierpopulation von Schritt b); und
d) Obduzieren jedes Säugetiers der Säugetierpopulation.

6. Verfahren nach Anspruch 5, wobei die Säugetierpopulation nach der Verabreichung einer Verbindung, von der vermutet wird, daß sie toxisch ist, an die Säugetierpopulation in Schritt b) über einen zweiten Zeitraum unter den in Anspruch 1 beschriebenen Aufstallungsbedingungen gehalten wird.

7. Verfahren nach Anspruch 6, wobei die Analyse gemäß Schritt c) mindestens einmal während dieses zweiten Zeitraums durchgeführt wird.

8. Verfahren nach Anspruch 6, wobei die Analyse gemäß Schritt c) mindestens dreimal während dieses zweiten Zeitraums durchgeführt wird, wobei die zweite und jede weitere Analyse nach einem Zeitraum durchgeführt wird, bei dem es sich um das Doppelte des zeitraums, der seit der vorhergehenden Analyse vergangen ist, handelt.

9. Verfahren nach Anspruch 8, wobei die erste Analyse sieben Tage nach Verabreichung der Verbindung in Schritt b) durchgeführt wird.

10. Verfahren nach einem der Ansprüche 5 bis 9, das weiterhin umfaßt, daß man das Körpergewicht, die Futteraufnahme, die Trinkflüssigkeitsaufnahme und klinische Symptome der Säugetierpopulation verfolgt.

11. Verfahren nach einem der Ansprüche 5 bis 10, das weiterhin umfaßt, daß man Abnormitäten für jedes Säugetier der Säugetierpopulation verfolgt.

12. Verfahren nach einem der Ansprüche 5 bis 11, wobei das Analysieren das Vergleichen des Metaboloms der Säugetierpopulation mit einer Referenz umfaßt.

13. Verfahren nach Anspruch 12, wobei das Vergleichen das Erstellen eines metabolischen Profils für das Metabolom eines Säugetiers der Säugetierpopulation und Vergleichen dieses Profils mit einer Referenz umfaßt.

14. Verfahren nach Anspruch 13, wobei ein Unterschied zwischen den metabolischen Profilen eine Verbindung, die das Metabolom eines Säugetiers beeinflußt, anzeigt.

15. Verfahren nach Anspruch 12, wobei Vergleichen das Vergleichen von mindestens einem Metaboliten des Metaboloms eines Säugetiers der Säugetierpopulation mit einer Referenz umfaßt.

16. Verfahren nach Anspruch 15, wobei ein Unterschied bezüglich des mindestens einen Metabolits eine Verbindung, die das Metabolom eines Säugers beeinflußt, anzeigt.

17. Verfahren nach einem der Ansprüche 5 bis 16, wobei es sich bei der Verbindung um eine Verbindung, von der vermutet wird, daß sie toxisch ist, oder eine Verbindung, von der vermutet wird, daß sie ein Arzneistoff ist, handelt.

18. Verfahren zum Identifizieren eines Markers für eine Verbindung, die das Metabolom eines Säugers beeinflußt, umfassend die Schritte des Verfahrens nach einem der Ansprüche 5 bis 17 sowie den weiteren Schritt des Bereitstellens eines Markers für diese Verbindung aufgrund der Analyse des Metaboloms.

19. Verfahren nach Anspruch 18, wobei es sich bei dem Marker um ein metabolisches Profil oder um mindestens einen Metaboliten eines Säugers der Säugetierpopulation, das/der im Vergleich zu einer Referenz verändert ist, handelt.

20. Verfahren nach Anspruch 18 oder 19, wobei der Marker die Toxizität einer Verbindung, die Wirkungsweise einer Verbindung oder eine therapeutische Wirkung einer Verbindung anzeigt.

21. Verfahren zum Identifizieren einer Verbindung, die das Metabolom eines Säugers beeinflußt, wobei das Verfahren die metabolische Analyse einer Probe von mindestens einem Säuger einer Säugetierpopulation, an die eine Verbindung, von der vermutet wird, daß sie das Metabolom eines Säugers beeinflußt, verabreicht worden ist und wobei die Säugetierpopulation vor und nach Verabreichung der Verbindung unter den in Anspruch 1 beschriebenen Aufstallungsbedingungen gehalten worden ist, umfaßt.

22. Verfahren nach Anspruch 21, wobei das Analysieren das Vergleichen des Metaboloms der Probe eines Säugers von dieser Säugetierpopulation mit einer Referenz umfaßt.

23. Verfahren nach Anspruch 22, wobei das Vergleichen das Erstellen eines metabolischen Profils für die Probe eines Tiers der Säugetierpopulation und Vergleichen dieses Profils mit einer Referenz umfaßt.

24. Verfahren nach Anspruch 23, wobei ein Unterschied zwischen dem metabolischen Profil eine Verbindung, die das Metabolom eines Säugers beeinflußt, anzeigt.

25. Verfahren nach Anspruch 22, wobei Vergleichen das Vergleichen von mindestens einem Metaboliten des Metaboloms der Probe einer Säugetierpopulation mit einer Referenz umfaßt.

26. Verfahren nach Anspruch 25, wobei ein Unterschied bezüglich des mindestens einen Metabolits eine Verbindung, die das Metabolom eines Säugers beeinflußt, anzeigt.

27. Verfahren nach einem der Ansprüche 21 bis 26, wobei es sich bei der Verbindung um eine toxische Verbindung oder einen Arzneistoff handelt.

28. Verfahren zum Identifizieren eines Markers für eine Verbindung, die das Metabolom eines Säugers beeinflußt, umfassend die Schritte des Verfahrens nach einem der Ansprüche 21 bis 26 sowie den weiteren Schritt des Bereitstellens dieses Markers aufgrund der Analyse des Metaboloms.

29. Verfahren nach Anspruch 28, wobei es sich bei dem Marker um ein metabolisches Profil oder um mindestens einen Metaboliten des Metaboloms der Probe eines Säugers der Säugetierpopulation, das/der im Vergleich zu einer Referenz verändert ist, handelt.

30. Verfahren nach Anspruch 28 oder 29, wobei der Marker die Toxizität einer Verbindung, die Wirkungsweise einer Verbindung oder eine therapeutische Wirkung einer Verbindung anzeigt.

## Revendications

1. Méthode d'obtention d'une population d'animaux mammifères présentant un métabolome essentiellement identique, ladite méthode comprenant :
a) le rassemblement d'une population d'animaux mammifères ayant essentiellement le même âge ;
b) la conservation de ladite population d'animaux mammifères de l'étape a) pendant une première période de temps suffisante à l'acclimatation dans les conditions d'hébergement suivantes :
i) température constante ;
ii) humidité constante ;
iii) séparation physique des animaux mammifères de la population d'animaux mammifères ;
iv) alimentation à volonté, où les aliments fournis ne contiennent essentiellement aucun contaminant chimique ou microbien ;
v) boisson à volonté, où le liquide fourni ne contient essentiellement aucun contaminant chimique ou microbien ;
vi) période d'illumination constante ; et
c) l'obtention de la population d'animaux mammifères de l'étape b) après ladite première période de temps.

2. Méthode conforme à la revendication 1, où ladite population d'animaux mammifères est une population de rongeurs.

3. Méthode conforme à la revendication 2, où ledit rongeur est un rat ou une souris.

4. Méthode conforme à la revendication 3, où ledit âge est compris dans l'intervalle X ± 1 jour, X étant l'âge envisagé pour la population.

5. Méthode d'identification d'un composé affectant le métabolome d'un animal mammifère, ladite méthode comprenant :
a) l'obtention d'une population d'animaux mammifères grâce aux étapes de la méthode de la revendication 1 ;
b) l'administration à ladite population d'animaux mammifères d'un composé soupçonné d'affecter le métabolome d'un animal mammifère ;
c) l'analyse du métabolome de la population d'animaux mammifères de l'étape b) ; et
d) la mise en oeuvre d'une nécropsie de chacun des animaux mammifères de la population d'animaux mammifères.

6. Méthode conforme à la revendication 5, où la population d'animaux mammifères est conservée pendant une seconde période de temps après administration à la population d'animaux mammifères d'un composé soupçonné d'être toxique dans l'étape b) dans les conditions d'hébergement décrites dans la revendication 1.

7. Méthode conforme à la revendication 6, où l'analyse de l'étape c) est mise en oeuvre au moins une fois pendant ladite seconde période de temps.

8. Méthode conforme à la revendication 6, où l'analyse de l'étape c) est mise en oeuvre au moins trois fois au cours de ladite seconde période de temps, la seconde analyse et toute analyse ultérieure étant mises en oeuvre après une période de temps double de la période de temps s'étant écoulée depuis l'analyse précédente.

9. Méthode conforme à la revendication 8, où la première analyse est mise en oeuvre sept jours après administration du composé de l'étape b).

10. Méthode conforme à l'une quelconque des revendications 5 à 9, comprenant en outre la surveillance de la masse corporelle, de la consommation alimentaire, de la consommation de liquide et des indicateurs cliniques de la population d'animaux mammifères.

11. Méthode conforme à l'une quelconque des revendications 5 à 10, comprenant en outre la surveillance des anomalies de chaque animal mammifère de la population d'animaux mammifères.

12. Méthode conforme à l'une quelconque des revendications 5 à 11, où l'analyse comprend la comparaison du métabolome de la population d'animaux mammifères à une référence.

13. Méthode conforme à la revendication 12, où la comparaison comprend la génération d'un profil métabolique pour le métabolome d'un animal mammifère de la population d'animaux mammifères et la comparaison dudit profil à une référence.

14. Méthode conforme à la revendication 13, où une différence de profil métabolique indique un composé affectant le métabolome d'un animal mammifère.

15. Méthode conforme à la revendication 12, où la comparaison comprend la comparaison d'au moins un métabolite du métabolome d'un animal mammifère de la population d'animaux mammifères à une référence.

16. Méthode conforme à la revendication 15, où une différence du ou desdits métabolites indique un composé affectant le métabolome d'un mammifère.

17. Méthode conforme à l'une quelconque des revendications 5 à 16, où ledit composé est un composé soupçonné d'être toxique ou un composé soupçonné d'être un médicament.

18. Méthode d'identification d'un marqueur pour un composé affectant le métabolome d'un mammifère, ladite méthode comprenant les étapes de la méthode conforme à l'une quelconque des revendications 5 à 17 et une étape supplémentaire d'obtention d'un marqueur pour ledit composé en se basant sur l'analyse du métabolome.

19. Méthode conforme à la revendication 18, où ledit marqueur est un profil métabolique ou est au moins un métabolite d'un mammifère de la population d'animaux mammifères altéré par rapport à une référence.

20. Méthode conforme à la revendication 18 ou 19, où ledit marqueur indique la toxicité d'un composé, un mode d'action d'un composé ou une activité thérapeutique d'un composé.

21. Méthode d'identification d'un composé affectant le métabolome d'un mammifère, ladite méthode comprenant l'analyse métabolique d'un échantillon d'au moins un mammifère d'une population d'animaux mammifères à laquelle un composé soupçonné d'affecter le métabolome d'un mammifère a été administré, ladite population d'animaux mammifères ayant été hébergée avant et après administration du composé dans les conditions d'hébergement décrites dans la revendication 1.

22. Méthode conforme à la revendication 21, où ladite analyse comprend la comparaison du métabolome de l'échantillon d'un mammifère de ladite population d'animaux mammifères à une référence.

23. Méthode conforme à la revendication 22, où la comparaison comprend la génération d'un profil métabolique pour l'échantillon provenant d'un animal de la population d'animaux mammifères et la comparaison dudit profil à une référence.

24. Méthode conforme à la revendication 23, où une différence de profil métabolique indique un composé affectant le métabolome d'un mammifère.

25. Méthode conforme à la revendication 22, où la comparaison comprend la comparaison d'au moins un métabolite du métabolome de l'échantillon d'une population d'animaux mammifères à une référence.

26. Méthode conforme à la revendication 25, où une différence du ou desdits métabolites indique un composé affectant le métabolome d'un mammifère.

27. Méthode conforme à l'une quelconque des revendications 21 à 26, où ledit composé est un composé toxique ou un médicament.

28. Méthode d'identification d'un marqueur pour un composé affectant le métabolome d'un mammifère, ladite méthode comprenant les étapes de la méthode conforme à l'une quelconque des revendications 21 à 26 et une étape supplémentaire d'obtention dudit marqueur en se basant sur l'analyse du métabolome.

29. Méthode conforme à la revendication 28, où ledit marqueur est un profil métabolique ou est au moins un métabolite du métabolome de l'échantillon provenant d'un mammifère de la population d'animaux mammifères, altéré par rapport à une référence.

30. Méthode conforme à la revendication 28 ou 29, où ledit marqueur indique la toxicité d'un composé, un mode d'action d'un composé ou une activité thérapeutique d'un composé.
